# EUROPEAN PATENT SPECIFICATION

(11) **EP 2 309 875 B1**
(45) Date of publication and mention of the grant of the patent: **21.03.2012**
(21) Application number: 08802983.0
(22) Date of filing: 08.08.2008
(51) Int. Cl.: C11B 1/10, A23L 1/20, A23D 9/007

(54) **METHOD FOR OBTAINING HIGHLY PURIFIED AND INTACT SOYBEAN HYPOCOTYLS**
VERFAHREN FÜR DIE GEWINNUNG VON HOCHAUFGEREINIGTEN INTAKTEN SOJABOHNENHYPOKOTYLEN
PROCÉDÉ D'OBTENTION D'HYPOCOTYLES DE SOJA PARFAITEMENT PURIFIÉS ET INTACTS

(43) Date of publication of application: 20.04.2011
(73) Proprietor: Alpro Comm. VA., 8560 Wevelgem (BE)
(72) Inventor: VANDENBROUCKE, Chris, Leo, Hilda, B-8200 St. Andries Brugge (BE); CALLEWAERT, Danilo, Frantz, B-8560 Wevelgem (BE); ARNOUT, Joost, Albert, Margriet, B-8900 Ieper (BE); NAIL, Jean-Noël, F-68530 Buhl (FR)
(74) Representative: Vanhalst, Koen
(86) International application number: PCT/EP2008/060473
(87) International publication number: WO 2010/015284

(56) References cited:
- WO-A1-02/37987
- JP-A- 10 276 748
- US-A- 5 952 230
- US-A1- 2002 081 365
- US-A1- 2004 219 282
- JUN YU, YUAN-FA LIU, AI-YONG QIU, YING-GUO WANG: "Preparation of isoflavones enriched soy protein isolate from defatted soy hypocotyls by supercritical CO2" LWT FOOD SCIENCE AND TECHNOLOGY, vol. 40, 2007, pages 800-806, XP002503641
- J.P. FELIX D'MELLO: "Handbook of Plant and Fungal Toxicants" 1997, CRC PRESS , XP002503642 page 127 - page 128
- HITOSHI SATO, KAZUKO ITO, KEIICHI SAKAI, YASUSHI MORINAGA, EIJI SUKEGAWA, TAKEHIKO KITAMURA, HIROYUKI SHIMASAKI, HIROSHIGE ITAKURA: "Effects of Soybean-germ Oil on Reducing Serum Cholesterol Level" JOURNAL OF OLEO SCIENCE, [Online] vol. 50, no. 8649, 2001, XP002506924 Retrieved from the Internet: URL:http://www.jstage.jst.go.jp/article/jo s/53/1/53_9/_article/-char/en> [retrieved on 2008-12-05]
- ANONYMOUS: "Phytosterols, LDL Cholesterol Levels and Atherosclerosis" NUTRITIONAL VALUE, BENEFITS AND RISKS OF SELECTED FOODS: FOOD & DISEASE, [Online] 2004, pages 1-2, XP002506925 Retrieved from the Internet: URL:http://www.goodwithchildren.com/food/l ipids/phytosterols.aspx> [retrieved on 2008-12-05]

## Description

### TECHNICAL FIELD

The present invention relates to the field of food technology and is directed to a method for obtaining highly purified and intact soybean hypocotyls. In particular, the present invention provides a method for separating a soybean material essentially comprising soybean germs and soybean cracks into a soybean germ concentrate having a predominant amount of undamaged germs. The invention also provides a soybean germ concentrate and uses thereof, e.g. for the preparation of a soybean germ oil and/or food products.

### BACKGROUND

Soybeans have been utilized for thousands of years as a source of both oil and protein. The bulk of the crop is solvent-extracted for vegetable oil and then defatted soybean meal is used for animal feed. A small proportion of the crop is consumed directly by humans. Soybean products do appear in a large variety of processed foods.

A raw soybean essentially comprises three main parts: an outer covering, referred to as the hull, two bean halves, also referred to as the meat or cotyledon; and a hypocotyl, also referred to as germ or embryo.

In conventional soybean processing, the hulls are often removed to facilitate oil extraction and to reduce the amount of fiber in the soybean meal. Typically, when the oil is produced from the soybean, foreign substances such as stem, sheath and other seeds are first removed from a starting soybean material in a selection step for improving quality of a final oil product and defatted cake. Then, the material is crushed by means of a crushing roller and hereby a mixture of hulls, cracks (= parts of broken cotyledons) and hypocotyls are formed. Most of the hulls, which contain components such as a pigment that will adversely affect the quality of oil, are generally removed by a series of aspirating and sifting steps, e.g. by using a vibrating sieve or a sorting apparatus with air. The cracks and hypocotyls are then flaked altogether to destruct their cell structures and to enable subsequent extraction of oil, followed by the extraction with n-hexane to give a crude oil, which is finally purified to yield the soybean oil. In such conventional process, some of the hypocotyl fraction is processed with the cotyledons, and some may be unintentionally aspirated off with the hulls.

Soybeans contain isoflavones such as genistein and daidzein, types of phytoestrogen, which are considered by some nutritionists and physicians to be useful in the prevention of cancer. Isoflavone concentration is not uniformly distributed throughout a soybean. The isoflavone content (on a weight percent basis) of the hypocotyl is typically at least about three times, and often ten times or more, the isoflavone content of the cotyledons. Consequently, some health food manufacturers have focused on the soybean hypocotyl as a source of isoflavones. Soybean hypocotyl concentrates may be produced and soybean hypocotyl concentrates may be extracted, leaving soybean hypocotyl oil and soybean hypocotyl meal.

Technical methods for producing such soybean germ concentrates have been described in the prior art. Typically, mechanical separation methods, using size, weight, and gravity differences between germs and cotyledons or parts of the latter, also denoted cracks, have been reported.

WO 96/10341 for instance discloses a method for processing soybeans to obtain essentially pure soybean germs comprising the steps of splitting cleaned soybeans followed by sifting of the obtained product to yield a fraction containing larger cotyledon pieces (cracks) and a second fraction containing smaller cracks, germs and hulls. The germs are then removed from this second fraction by means of separation steps based on differences in floating behavior in air.

Another example of a processing technique is disclosed in EP 1,142,489 which reports a method for processing soybeans comprising cleaning soybeans, splitting the cleaned beans, and further processing of the split beans, e.g. flaking and preparation of oil. In the disclosed method crushed beans are separated with a separating apparatus such as a sieve and a sorting apparatus with air. A fraction containing hulls and germs as main components is separated and collected by sorting with air. From this fraction a hull part is removed by sorting with air and a remaining germ part is concentrated. The method disclosed in EP 1,142,489 is thus directed to the purification of germs from a mixture of aspirated hulls and germs.

WO 02/37987 discloses a method of producing a soy germ concentrate comprising cracking whole soybeans such that about 50% of the cracked particles are larger than 3.35 mm. Next, soy germ is separated from this cracked soybean stream (which contains soybean meats, germ and hulls) by passing it through an initial sifting machine which separates the larger meat and hull pieces from the stream, and further sifting the stream so that the germs are separated from the meats based on the respective sizes of the germs and meats.

In US 2004/0219282 and US 2002/0081365, a method is described for producing a germ-enriched soybean material, wherein the whole soybeans are crudely crushed, into the size of less than ½, followed by sieving and sorting.

A mechanical separation method for isolating soybean germs of over 90% purity is disclosed in US 5,952,230. The method comprises processing soybeans according to a conventional split/crush process, after which the soybean mixture is passed through a peeling process to separate it into split soybeans and a mixture of hulls and germs. This mixture is sifted to further increase its germ content. The germs are finally separated from the hulls by gravity force, e.g. by introducing this germ-enriched mixture into a cyclone.

In view of the above properties of soybean germs there is a need in the art for methods permitting to separate soybean germs from the remaining parts of soybeans and to obtain pure and undamaged soybean germs. However, frequently encountered problems with prior art methods include high manufacturing cost & investments, low amounts of recovered germs, loss and/or destruction of nutrients in the germs due to damaged germs, recovery of germs comprising too much impurities, etc..

In view of the above, it is clear that there remains a need in the art for an improved method for separating soybean germs which overcomes at least some of the above-mentioned problems.

### SUMMARY

The present invention is directed to a method for separating essentially pure and undamaged soybean hypocotyls from a soybean product comprising a mixture of cotyledons, cracks, hulls and germs. More in particular, the invention provides a method for separating soybean germs from a mixture essentially comprising soybean cracks and soybean germs based on a difference in form between said soybean cracks and soybean germs.

In a first aspect, the invention provides a method for processing soybeans as defined in claims 1 to 7.

The method gives a good separation between cracks and germs without damaging the germs such that essentially pure and undamaged soy germs can be retrieved according to the method. Moreover, the method also enables the separation of germs from small cracks having about the same size and weight as the soy germs. Having regard to the prior art in the present technical field of processing whole soybeans, the method can be considered as unconventional and unusual, especially since the prior art in this field typically teaches a different approach for separating a mixture of soybean products including cotyledons, cracks, hulls and germs, i.e. by conventional separation apparatuses using vibrating sieves and/or sorting apparatuses with air, which all operate based on density differences (e.g. differences in weight and/or size) of the material to be separated. In addition, the method goes beyond what is thought as feasibly possible by prior art techniques, since where a fraction comprising soybean cracks and soybean germs having about the same density can no longer be separated using conventional techniques, contrary to what is done in the prior art, the method provides for an additional separation of this material. Thus, unusually the method permits to extract relatively more germs from soybean material compared to prior art techniques.

The soybean material essentially comprising cracks and germs can be obtained in various ways.

The soybean material essentially comprising germs and cracks obtained by the steps of:

### Method a:

a1) Separating by moderate aspiration (11a) the soybean product (2) into a hull fraction (3a) essentially comprising hulls and a dehulled fraction (4a) essentially comprising cotyledons, cracks and germs, and
a2) separating said dehulled fraction into a cotyledon-rich fraction (8) and a soybean material essentially comprising soybean cracks and germs, by subjecting said dehulled fraction to one or more sifting and/or aspirating steps (12a). In this step some remaining hulls can still be removed.

Applying method a, the main part of the soybean material (5) is originating from the dehulled fraction (4a). For efficiency or yield reasons, separating (extra) soybean material (5) from the hull fraction (3a) can be performed using sifting and/or aspiration (13a).

Alternatively, a second method for obtaining a soybean material essentially comprising germs and cracks comprises the steps of:

### Method b:

b1) Separating by strong aspiration (11 b) the soybean product (2) into a hull fraction (3b) essentially comprising hulls, germs and some smaller cracks and a dehulled fraction (4b) essentially comprising cotyledons and cracks, and
b2) Separating hulls from said hull fraction (3b) by subjecting said hull fraction to one or more separation steps (13b) based on a difference of size and/or weight between said hulls and said smaller cracks and germs.

Applying method b, the main part of the soybean material (5) is originating from the hull fraction (3b). For efficiency or yield reasons, separating (extra) soybean material (5) from the dehulled fraction (4b) can be performed using sifting and/or aspiration (12b). For reasons of ease of production, less investments and better results, method a is to be preferred to method b.

Both above-disclosed thus yield a soybean material essentially comprising cracks and germs, which have about a same size and weight. Moreover, the applied separation techniques, which are based on differences in size and/or weight between the materials to be separated, usually do not significantly damage the soybean germs. The method further permits to retrieve, concentrate, and thus valorize soybean germs originating from different separation fractions in a simple and efficient way. Moreover, unexpectedly the germs which do not show considerable differences in size and/or weight compared to cracks in this fraction can be further separated using the method step mentioned above.

The whole soybeans can be heated before being broken preferably to a temperature of between 60°C and 80°C for between 1 and 10 min. Such pre-treatment permits to facilitate subsequent splitting and dehulling of the soybeans, without generating too much cracks and without damaging the soybean germs.

The soybean germs obtained in accordance with the method disclosed herein can be further processed. In one example hereof, said soybean germs are subjected to supercritical CO₂ extraction and soybean germ oil is prepared from said extracted soybean germs. The method thus retrieves soybean germ oil from a soybean germ product predominantly comprising undamaged soybean germs that have retained their nutritional value, yielding a germ oil of improved organoleptic and nutritional quality. Furthermore, the applied technique for preparing the soybean germ oil permits to preserve the nutritional quality of the soybean germs, and is completely non-toxic.

In another aspect, the invention relates to a soybean germ concentrate according to claims 11 or 12 and uses thereof according to claim 17.

In yet another aspect, the invention relates to a soybean germ oil according to claims 13 to 16 and uses thereof according to claim 18, e.g. for the preparation of a food product. Disclosed herein is a soybean germ oil having an amount of linolenic acid (C18:3) of between 10 and 30 wt %, preferably between 12 and 28 wt%, more preferably between 15 and 25 wt%, most preferably between 18 wt% till 23 wt% on total fatty acids wt%. The soybean germ oil can be prepared from a soybean germ concentrate as defined herein. Further disclosed herein is a soybean germ oil with a high concentration of phytosterols and/or tocopherols and/or linolenic acid %. In particular, a soybean germ oil having an amount of soybean phytosterols of at least 5%, preferably 6%, more preferably 7%, even most preferably between 5.0 and 7.2%. Also disclosed herein is a soybean germ oil having an amount of soybean tocopherols of at least 0.10%, more preferably at least 0.20%, most preferably at least 0.25% and preferably less than 0.40%. Furthermore, the disclosure also relates to a soybean germ oil substantially free of lecithin.

Adding back soybean phytosterols to cholesterol reducing functional foods carrying a health claim (such as margarines for example) is well known in the art (cfr. Benecol). These commercially available soybean phytosterols are normally obtained during the refining of hexane extracted standard crude soybean oil. They can be found in the volatiles obtained during the steam deodorization stage from which they can be isolated by condensation.

After drying, the soybean germ oil obtained by supercritical CO₂-extraction from the highly pure and intact soy germ concentrate produced according to the method disclosed herein does not contain the soybean phosphatides ("lecithin") and can be considered as pre-refined ("degummed") and naturally contains very high amounts of soybean phytosterols (e.g. 7.2%) and soybean tocopherols (e.g. 0.26%).

The disclosure also relates to soybean germ meal and uses thereof, e.g. for the preparation of a food product, a food supplement, a pharmaceutical or cosmetic product. The invention provides a soybean germ meal comprising a wt% of isoflavones higher than 2.0%, preferably higher than 2.5%. In a particular embodiment the soybean germ concentrate further comprises saponins higher than 2.6 wt%, preferably higher than 3.2%.

In still another aspect, the invention also relates to the use of a soybean germ concentrate or of a soybean germ oil as defined herein in a food product, and to a food product comprising a soybean germ concentrate as defined herein.

With the insight to better show the characteristics of the invention, some preferred embodiments and examples are described hereafter referring to the enclosed drawings.

### Description of the figure

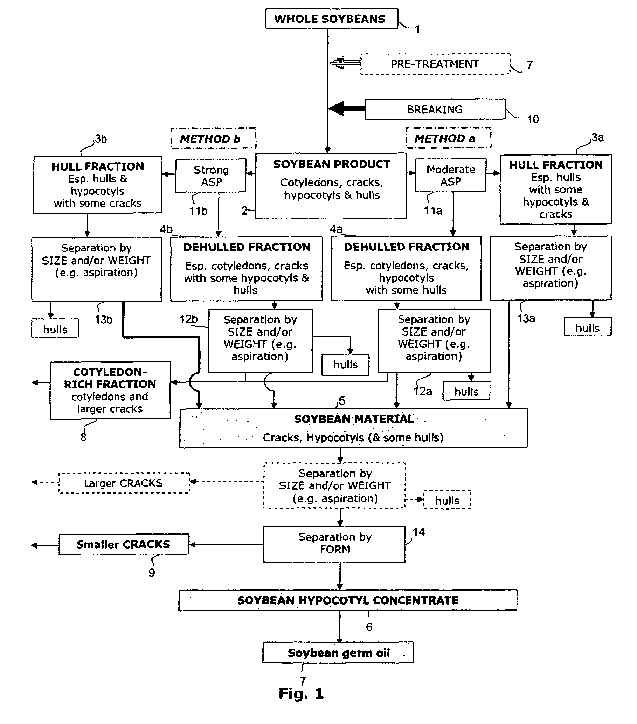

**FIG. 1** is a flow chart showing steps of a method as disclosed herein.

### Detailed description of the invention

### METHOD

The present invention relates to a method for processing soybeans (Glycine *max*). It shall be noted that the terms "***soy***" and "***soybean***" and "***soya***" are used herein as synonyms.

The major parts of a soybean include an outer covering, herein referred to as the hull; two bean halves, herein referred to cotyledon or endosperm; and an embryo or germ, herein referred to as the hypocotyl. The hull of the mature bean is hard and protects the cotyledon and germ from damage and subsequent (esp. oil) oxidation.

The term "***whole soybeans***" in this context refers to soybeans that have not yet undergone a breaking process and that comprise all elements of the beans, thus including hull; two bean halves, and germ.

The term "***cotyledon***" as used herein is intended to refer to an undamaged bean halve.

The term "***crack***" or "***soybean crack***" as used herein is intended to refer to pieces of cotyledons. This term thus also includes damaged bean halves and pieces of cotyledons which are smaller than a bean halve. Cracks generally consist of relatively randomly and irregularly shaped pieces of cotyledons, which are obtained after fracturing or splitting of the cotyledons.

In reference to **FIG. 1**, a method provided comprising the steps of:
a) separating a soybean hypocotyl concentrate (6) from smaller soybean cracks (9) out of soybean material (5), which separation is based on a difference in form (14) between said soybean hypocotyls and said soybean cracks,
b) enriching said soybean material (5), mainly comprising soybean cracks and soybean germs, by removal of soybean hulls, soybean cotyledons, and the larger soybean crack fraction from the soybean product (2) using traditional separation methods based on differences in size and/or weight (e.g. aspiration),
c) obtaining the soybean product (2) by breaking (10) the whole soybeans (1) in a limited number of parts, such as cotyledons, cracks, hulls and hypocotyls.

Optionally, in a still earlier step, the starting material, i.e. whole soybeans, is first cleaned by removing foreign substances such as stem, sheath, weeds, sands, metal particles and small stones, before being broken.

In another optional step, the soybeans may be pretreated (7) in order to facilitate their downstream processing. Pre-treatment may comprise the heating of said whole soybeans to a temperature of between 60°C and 80°C for between 1 and 10 minutes; e.g. in order to avoid abundant crack formation during downstream processing and esp. during breaking.

Next, the whole soybeans are broken according to **step c)** of the above method. Breaking (10) can be done by means of any known apparatus making use of friction, impact stress, shearing stress and the like. For instance, soybeans can be split by means of a crushing roller or rubber roller or the like. Typically for soybean oil crushing purposes, soybeans are broken on average into 8 "equally sized" pieces (cracks). The whole soybeans can be split in two halves after preheating the soybeans and by using an impact mill in order to avoid or at least reduce loss (dust), cell damage, destruction on and/or damaging of soybean cotyledons and germs. As a result of the breaking step a soybean product is obtained which comprises a mixture of cotyledons, cracks, hulls and hypocotyls (and a very minor fraction of whole soybeans can stay present too).

A next **step b)** of the above method comprises enriching from said soybean product a soybean material essentially comprising soybean cracks and soybean germs. Methods for preparing such soybean material will be further detailed below.

**Step a)** of the above defined method is then directed to the separation of soybean germs from said soybean material (5). More specifically, said step comprises the separation of soybean germs from soybean cracks having about a same density, i.e. a same weight and/or size. The present method for processing whole soybeans comprises the step of separating a soybean material (5) essentially comprising cracks and germs into a fraction rich in cracks (9) and a fraction rich in soybean germs (6), also denoted herein as a soybean hypocotyl concentrate. The present separation method is based on differences in form between soybean germs and the soybean cracks from which they are to be separated. The difference in form also results in a difference in slipperiness or smoothness.

For separating soybean germs and soybean cracks which are of a similar size and/or weight prior art using separation techniques based on difference in size and/or weight have up to now not been successful. The inventors have now surprisingly shown that differences in form (and the resulting slipperiness) between such cracks and germs can be used for effectively separating soybean cracks from soybean germs. In particular, in contrast to techniques used in the prior art, the inventors have developed a separation technique which does not require a difference in density between the components that are to be separated. Furthermore, the present separation technique does not require vibrating and/or oscillating movements to obtain separation of germs from cracks.

The term "***difference in form***" as used herein refers to the fact that soybean germs and soybean cracks of about a same size and/or weight show differences in form. Differences in form for instance comprise the fact that undamaged germs generally are more elongated and have a smoother surface than cracks, which show many flat faces (by fracture) on their surface. As a result, undamaged germs are much more slippery than cracks. Sometimes the more elongated form of the undamaged germs is referred to as a 'banana' form.

More in particular the method disclosed herein comprises the step of subjecting said soybean material to a centrifugal force to separate said soybean material in a fraction rich in cracks and a fraction rich in soybean germs. This separation step can be practically carried out by means of an indented cylindrical device.

The term "*indented cylindrical device*" as used herein is intended to refer to a device comprising a cylindrical drum which is indented and capable of rotating. The term "*cylindrical drum*" in this context is to be understood in its broadest sense, and not only comprises drums having an essentially constant diameter but also drums having a diameter that may vary along the length of said drum. For instance such drum may be a cylindrical, conical, polygonal drum or a drum having other forms of variable curvature.

It shall be noted that the centrifugal or rotating force in this context, applied by means of an indented cylindrical device, is important as the Applicants have noted that separation using vibration or oscillation techniques which are based on density differences between the materials to be separated turned out to be less efficient, especially in view of the fact that the differences in density between the components to be separated is minor.

In one example said indented cylindrical device corresponds to an "indented cylinder". Indented cylinders are apparatuses having a rotating cylinder and a movable separating trough. Mostly the rotating speed of the cylinder can be varied. The inside surface of the cylinder has small indents. The indents are given a design by which ideally one single particle is to be received in each pocket. Indented cylinders operate on the centrifugal force principle by which the speed of the cylinder holds particles in the indents lifting them out of the mass of particles until the indent is inverted to the point where the gravity causes the lifted particles to fall out of the indent. Separation happens when these lifted particles fall into the separating trough.

The cylindrical device should be rotated with such a speed that the germs and cracks will be held in the indents by means of gravity and centrifugal force for a time long enough to perform a good separation. The (undamaged) hypocotyls have a banana-like form, which is quite slippery, and fall back into the cylinder, whereas cracks stay longer into the indents, are more lifted up, and have a bigger chance to fall into the gully or trough, of which the position can be adjusted. Therefore, the method comprise subjecting said soybean material to a centrifugal force of at least 0.1 times gravity force, and preferably of at least 0.2 times g-force. In another example the indented cylindrical device may be rotated at a speed comprised between 20 and 60 rpm.

In another example, the indents have a form and shape for temporarily housing soybean hypocotyls. They are usually ball-like shaped, with a diameter of about 3 mm. Preferably the indents are drop shaped, since this results in a more easy and better separation. The drop shape of the indent (one side of the indent has an angle of about 45°: easy to fall in; the other side has an angle of nearly 90°: difficult to escape) holds the cracks very well into the indents. The (smaller) cracks, usually having dimensions of 1 mm by 2.5mm by 2.5mm (so with a lot of fracture surfaces & non-elongated) can totally fall into the indent. The cracks can only escape from the indents at a certain height, and mostly they fall into the trough. On the other hand, undamaged hypocotyls, having dimensions of about 4 mm by 1.5mm by 2 mm, can fall into the indents, but they easily slide out of the indent because of their form (slippery) and elongation.

Practically, in accordance with the method disclosed herein the soybean material essentially comprising soybean cracks and soybean germs can be fed into the apparatus through a conveyor into the center of the rotating cylinder. As the cylinder rotates the germs and cracks fall out of the indents at different axial positions (or angles or heights) due to gravity.

The present disclosure thus provides a method for obtaining a high amount of essentially undamaged soybean germs. The method for separating soybean germ in high purity as disclosed herein has the important advantage that germs can be separated at a high yield and/or concentration compared to conventional methods, and that a large quantity of soybean germs can be separated at a time. Moreover, another important advantage is that germs are separated by a simple process using a method which does not involve the destruction of functional nutrients in the germs, thereby enabling the retention of components in native state in the germs.

The present disclosure also relates to a method comprising the step of c) separating said soybean germs (6) from said soybean material (2) which separation is based on a difference in form between said soybean germs and said soybean cracks

The separated soybean cracks (9) and the soybean germs (6) can both be separately further processed.

Processing of the soybean germs may involve but is not limited to toasting, steaming, roasting, milling, agglomerating, coating, flaking, extraction or any combinations thereof. At the same time valuable flavors may develop. This is especially the case if the soybean germs are roasted.

In one example, the soybean germs can be toasted and ground to form soybean flour. For instance, soybean germs can be ground or milled to produce for instance a dry powder or flour which could then be either blended or used separately as a dietary supplement in a variety of animal or human food products.

In another example the soybean germs can be first solvent extracted to extract the soybean germ oil and the resulting defatted soybean germ meal can be used as a starting material for soy isoflavone and/or soy saponin production.

In a particular example, extraction of the soybean germs involves subjecting the soybean germ concentrate to a supercritical CO₂ extraction and to prepare a soybean germ oil from the extracted soybean germs. Because large volumes of soybean germs can be obtained using the process described above, it is estimated that commercial quantities of soybean germ oil can also generally be produced at a commercially viable cost.

### Soybean material and preparation thereof

In accordance with step b) of the method disclosure herein a soybean material that essentially comprises soybean germs and soybean cracks is provided herein. The term "***essentially comprising***" in this context is intended to refer to a soybean material which contains cracks and germs which in combination make up more than 80%, preferably more than 85, 90, or 95 % by weight of the total amount of the soybean material. This soybean material can be obtained in various ways, as is illustrated on **FIG. 1**.

In one example, the soybean material essentially comprising germs and cracks is obtained by the steps of:

### Method a:

a1) Separating by moderate aspiration (11a) the soybean product (2) into a hull fraction (3a) essentially comprising hulls and a dehulled fraction (4a) essentially comprising cotyledons, cracks and germs, and
a2) separating said dehulled fraction into a cotyledon-rich fraction (8) and a soybean material essentially comprising soybean cracks and germs, by subjecting said dehulled fraction to one or more sifting and/or aspirating steps (12a). In this step some remaining hulls can still be removed.

Applying method a, the main part of the soybean material (5) is originating from the dehulled fraction (4a). For efficiency or yield reasons, separating (extra) soybean material (5) from the hull fraction (3a) can be performed using sifting and/or aspiration (13a).

Alternatively, a second example relates to a method for obtaining a soybean material essentially comprising germs and cracks by the steps of:

### Method b:

b1) Separating by strong aspiration (11b) the soybean product (2) into a hull fraction (3b) essentially comprising hulls, germs and some smaller cracks and a dehulled fraction (4b) essentially comprising cotyledons and cracks, and
b2) Separating hulls from said hull fraction (3b) by subjecting said hull fraction to one or more separation steps (13b) based on a difference of size and/or weight between said hulls and said smaller cracks and germs.

Applying method b, the main part of the soybean material (5) is originating from the hull fraction (3b). For efficiency or yield reasons, separating (extra) soybean material (5) from the dehulled fraction (4b) can be performed using sifting and/or aspiration (12b).

For reasons of ease of production, less investments and so far better results, method a is to be preferred to method b.

Using prior art techniques soybean germs will generally be contained in the fraction comprising the hulls. The methods disclosed herein not only enrich soybean germs from a dehulled soybean fraction, but in addition enrich soybean germs and soybean cracks from said hull fraction, which have - sometimes unintentionally- been aspirated with hulls and are containing within the hull fraction.

### Separated hull fraction and dehulled fraction

Separation of a soybean product (2) comprising cotyledons, cracks, hulls and germs into a hull fraction (3a/b) and a dehulled fraction (4a/b) includes the removal of (mainly) hulls from the soybean product. Separation is preferably performed by aspiration, using any suitable means known to those skilled in the art such as an aspirator or pneumatic cleaner for example.

The hull fraction essentially comprises hulls and residual amounts of (smaller) cracks and germs which have been aspirated together with the hulls. In this context the term "*essentially comprising hulls*" is intended to refer to a fraction having an amount of hulls which is more than 75%, preferably more than 80% or even more than 85% by weight of the total amount of the fraction. The amount of smaller cracks and germs in the hull fraction therefore amount up to maximum 25%, and preferably is lower than 20% or 15% by weight of the fraction.

The dehulled fraction "*essentially comprising cotyledons, cracks and germs*" (containing germs particularly in case of method a) refers to a fraction which contains cotyledons, cracks and germs which in combination make up more than 80%, preferably more than 85, 90, or 95 % by weight of the total amount of this fraction.

In subsequent steps of the method disclosed herein the germs (and the smaller cracks) contained in the hull fraction and/or in the dehulled fraction are further separated, purified and concentrated.

### Processing of the dehulled fraction

In one example, the method disclosed herein comprises the separation and purification of germs which are contained in the dehulled fraction. Since this is most important for method a, we will focus on this method in this paragraph. More in particular, the method involves the step of separating the dehulled fraction (4a) into a cotyledon-rich fraction (8) and a soybean material (5) essentially comprising soybean cracks and germs by subjecting said dehulled fraction (4) to one or more sifting and/or aspirating steps. The present step can be carried out by using separating apparatuses such as sieves and/or sorting apparatuses with air by making use of differences in the gravity and density between cotyledons, cracks, and germs.

The "cotyledon-rich fraction" as used herein refers to a fraction essentially comprising cotyledons and residual amounts of larger cracks which have unintentionally been separated together with the cotyledons. The term cotyledon-rich fraction "*essentially comprising cotyledons*" is intended to refer to a fraction which contains more than 80%, preferably more than 85, 90, or 95 % by weight of the total amount of this fraction of cotyledons. This fraction can be further processed to form e.g. soy flour.

### Processing of the hull fraction

In another example, the method disclosed herein comprises the separation and purification of germs which are contained in the hull fraction. Since this is most important for method b, we will focus on this method in this paragraph. More in particular, the method involves the step of separating hulls from said hull fraction (3b) by subjecting said hull fraction to one or more sifting and/or aspirating steps. In analogy with the above, the present step can also be carried out by using separating apparatuses such as sieves and/or sorting apparatuses with air by making use of differences in the gravity and density between hulls on one hand and cracks and germs on the other hand.

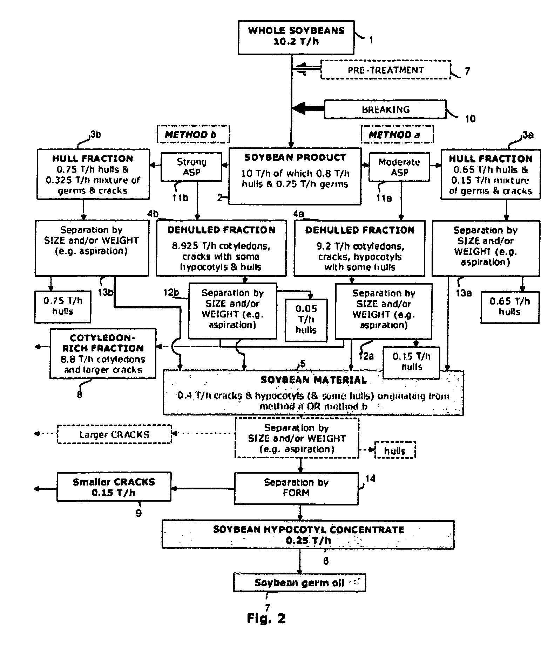

**FIG. 2****.** shows an example of a material balance and shows some product flows. This figure should help to correctly estimate the impact of our invention.

### PRODUCTS

The disclosure further relates to products that are obtained or obtainable by carrying out at least some steps of the method disclosed herein.

In a first example, the disclosure relates to **soybean material** essentially comprising soybean cracks and soybean germs, and especially comprising more than 80 % by weight based on the total amount of said material of cracks and germs. From this material soybean germs are further enriched according to the method disclosed herein. Said soybean material is obtained or obtainable by carrying out at least steps some steps of the above defined method.

In another example, the disclosure relates to a soybean germ concentrate comprising more than 85%, pref. more than 90%, more pref. more than 95% by weight based of soybean germs, of which preferably at least 60%, pref. more than 70%, more pref. more than 80% and most pref. more than 90% by weight thereof are essentially undamaged soybean germs. Soybean germs as disclosed herein have good nutritional and/or organoleptic properties, especially in view of their relatively "undamaged" character. Being damaged, the soybean germ oil would come into contact with air and suffer from oxidation, resulting in a product or germs with inferior organoleptic and/or nutritional properties.

With "***undamaged germs***" as used herein is meant intact germs which have not been broken. The (undamaged) hypocotyls have a banana-like form, which is quite slippery and elongated. Undamaged germs can be recognized by visual inspection and e.g. by the presence of a "seam", which is visible as a thin line extending over the substantially complete length of the germ.

The method disclosed herein permits to obtain a soybean germ concentrate having more than 85 wt%, and preferably more than 90 wt% or even more than 95 wt% of soybean germs, of which more than 60 wt%, and preferably more than 70 or even 80 or 90 wt% is undamaged.

Said soybean germ concentrate is obtained or obtainable by carrying out the method disclosed herein.

The disclosure also relates to the use of a soybean germ concentrate as defined herein in food products. The soybean germs as defined herein may form the basis of or be the active ingredient in various types of food products. The term "*food product*" as used in a broad sense in this context refers, and covers food for humans as well as food for animals (i.e. a feed). The food is preferably for human consumption. The food may be in the form of a solution or as a solid, depending on the use and/or the mode of application and/or the mode of administration. Non limitative examples of food products in which a soybean germ concentrate as defined herein may be used (after appropriate heat treatment and/or grinding) include for instance-bakery products, cookies, pasta products, snacks, nutritional bar products, but also nutritional beverages, or drinks.

In an example soybean germs can be roasted in hot air of 200 to 230 °C. The thus obtained roasted germs may be used as ingredient for breakfast cereals, candy bars, bakery products and teas. In another example; soybean germs may be dried and pulverized to obtain a powder for use in various food and health products, homeopathic products, veterinary products, fitness products, pet feed supplements. As a further alternative the germs may be extracted, puffed, swollen, hydrolyzed and/or fermented for use in food applications.

For instance, in yet another example, soybean germs can be extracted with organic solvents, such as hexane, ethanol, isopropanol and the like or combinations thereof or with more natural solvents, such as supercritical CO₂, to yield soybean germ oil. The resulting soybean germ oil contains a lot of (concentrated) phytonutrients or other interesting components. The soybean germ oil as defined herein (after extraction of the soybean hypocotyl concentrate using hexane or SC CO₂) comprises between 5% and 7.2% total phytosterols (by weight), preferably between 6% and 7.2% total phytosterols (by weight). Total phytosterols include beta-sitosterol, stigmasterol, Δ7-stigmasterol, campesterol, brassicasterol, Δ7-avenasterol, citrostadienol, cholesterol, a.o.

The soybean germ oil comprises between 18% and 23% linolenic acid on total fatty acids (by weight), preferably between 19%; 20%; 21%; 22% and 23% linolenic acid on total fatty acids. Usually this soybean germ oil comprises a higher concentration of total phytosterols as well as linolenic acid compared to soybean germ oils from the prior art. For methods to measure these compounds, we like to refer again to EP 1,142,489. Phytosterols (and -stanols) are nowadays famous for their health effects, and esp. famous because of their LDL-cholesterol lowering effects (esp. with obese species). Linolenic acid is one of the most unsaturated fatty acids, and oils with a high proportion of these unsaturated acids, are thought to be beneficial for health.

The soybean oil as defined herein is substantially free of lecithin.

Said soybean germ oil is obtained or obtainable from a soybean germ concentrate as defined herein.

The disclosure further relates to the use of such a soybean germ oil as defined herein in a food product as defined above. The disclosure also relates to food products comprising a soybean germ concentrate or a soybean germ oil as defined herein.

The disclosure further relates to a soybean germ oil and uses thereof, e.g. for the preparation of food products and/or food supplements and/or cholesterol lowering products and/or cosmetics and/or pharmaceuticals and/or nutraceuticals.

After oil extraction from the soybean hypocotyl concentrate, which was preferably milled, ground and/or crushed on beforehand, a residue product is obtained. This residue product is called **soybean germ meal**. Since soybean germs contain about 10 wt% soybean germ oil (which is essentially isoflavone-free), the residue of soybean hypocotyl meal is about 90% of the initial soybean germ weight. Thanks to the high concentration (highly purified) and intactness of the soybean hypocotyls in the soybean hypocotyl concentrate, the latter may comprise high concentrations of isoflavones (aglucones) and saponins. Consequently, the de-oiled soy germ meal comprises a wt% of isoflavones higher than 2.0%, pref. higher than 2.5%, but pref. lower than 3.5%. Furthermore, the soybean germ meal comprises a wt% of isoflavone aglucones higher than 1.1%, pref. higher than 1.4%. Moreover, this soybean germ meal comprises a wt% saponins higher than 2.6%, pref. higher than 3.2%, but pref. lower than 4.5%.

This de-oiled soybean germ meal is much more stable against oxidation, since the sensitive oil component had been removed substantially.

The disclosure further relates to a soybean germ meal and uses thereof, e.g. for the preparation of food products and/or food supplements and/or cholesterol lowering products and/or cosmetics and/or pharmaceuticals and/or nutraceuticals.

The present invention will be described in greater detail below with the aid of an example which follows. It goes without saying, however, that this example is given by way of illustration of the invention and does not constitute in any manner a limitation thereof.

### EXAMPLES

### Example 1: preparation of soy germ concentrate

This example illustrates a method according to the invention.

Whole soybeans were carefully selected and contaminants such as stems, stones, etc. were removed. The cleaned soybeans were then heated for 5 minutes till about 75°C, and subsequently broken by an impact mill into a size of about 1/2 of the original one (into cotyledons or halves) to give a mixture of hulls, germs, cracks and cotyledons. The hull part was then removed from the mixture by means of moderate aspiration (method a), and a dehulled part predominantly yielding cotyledons, cracks and germs is separated.

This dehulled fraction was further enriched in smaller cracks and germs, through separation of cotyledons (and the larger cracks) by sieving and remaining hulls by aspirating. A mixture of embryos and small cracks, having about a same density, are retrieved from the dehulled part when the latter is separated by size/weight/gravity difference.

An indented cylinder of Westrup A/S, TR-620, having a cylinder mantle (diameter of 0.6m) with drop shaped indents (diameter of about 3 mm), was operated at a rotating speed of 40 rpm. This rotation resulted in a centrifugal force of about 0.537 times gravity force.

After one run (passage) trough the indented cylinder, the soybean hypocotyl concentrate (taken from the cylinder mantle) had a purity of 93% (on weight basis) and an intactness of 83.4% (on 25 g total weight of the concentrate, 20.85 g were intact hypocotyls).

### Example 2: preparation & composition of soybean hypocotyl oil & meal

This example illustrates the preparation & composition of soybean germ oil. This soybean germ oil was extracted with hexane or with supercritical CO₂ from the soybean hypocotyl concentrate from Example 1.

The soybean germ oil, that had been extracted with hexane, comprises 5.2 % total phytosterols (by weight) and 21.3% linolenic acid (C18:3) on total fatty acids (by weight). The oil contains also other fatty acids such as C16:0 (13.8%); C18:0 (3.5%); C18:1 (7.2%); C18:2 (53.5%); and remaining fatty acids (0.7%) on total fatty acids (by weight). The oil contains also 2230 ppm (by weight) total tocopherols.

Before extraction, germs were milled below a diameter of 750 µm. The soybean germ oil, that had been extracted (for 6 h) with supercritical CO₂ (250 bar; 40 °C), comprises 7.2 % total sterols (by weight) and 22.1% linolenic acid (C18:3) on total fatty acids (by weight). The oil contains also other fatty acids such as C16:0 (13.1%); C18:0 (3.3%); C18:1 (7.4%); C18:2 (53.2%); and remaining fatty acids (0.9%) on total fatty acids (by weight). The oil contains also 2620 ppm (by weight) total tocopherols. Further this oil is substantially free of lecithin.

The soybean germ meal fraction comprised 2.64% isoflavones (of which 1.48% isoflavone aglucones) and 3.42% saponins.

## Claims

1. Method for processing whole soybeans comprising the steps of:
a) breaking whole soybeans in a soybean product (2) comprising cotyledons, cracks, hulls and germs,
b) enriching from said soybean product (2) a soybean material (5) essentially comprising soybean cracks and soybean germs, and
c) separating said soybean germs (6) from said soybean material (5) which separation is based on a difference in form between said soybean germs and said soybean cracks and wherein said separation step is carried out by means of an indented cylindrical device.

2. Method according to claim 1, wherein said soybean material (5) comprises more than 80% by weight of soybean cracks and soybean germs based on the total amount of said material.

3. Method according to claim 1 or 2, wherein said soybean material (5) is obtained by the steps of:
a1) separating by moderate aspiration (11a) said soybean product (2) into a hull fraction (3a) essentially comprising hulls and a dehulled fraction (4a) essentially comprising cotyledons, cracks, and germs, and
a2) enriching from said dehulled fraction (4a) said soybean material (5) by one or more sifting and/or aspirating steps.

4. Method according to claim 3, further comprising enriching from said hull fraction (3a) said soybean material (5) by one or more sifting and/or aspirating steps.

5. Method according to claim 1 or 2, wherein said soybean material (5) is obtained by the steps of:
b1) separating by strong aspiration (11b) said soybean product (2) into a hull fraction (3b) comprising hulls, germs and cracks and a dehulled fraction (4b) essentially comprising cotyledons and cracks, and
b2) enriching from said hull fraction (3b) said soybean material (5) by one or more sifting and/or aspirating steps.

6. Method according to any of claims 1 to 5, wherein said whole soybeans are heated to a temperature of between 60 and 80 °C for between 1 and 10 min before being broken.

7. Method according to any of claims 1 to 6, further comprising the step of subjecting said soybean germs (6) to supercritical CO₂ extraction and preparing soybean germ oil and soybean germ meal from said extracted soybean germs.

8. Use of an indented cylindrical device for separating soybean material into a soybean hypocotyl concentrate and soybean cracks.

9. Use of an indented cylindrical device according to claim 8, wherein the cylinder drum of said indented cylindrical device comprises a cylindrical mantle having a form and shape for temporarily housing soybean hypocotyls.

10. Use of an indented cylindrical device according to claim 8 or 9, wherein said soybean material is subjected to a centrifugal force of at least 0.1 times gravity, preferably of at least 0.2 times gravity.

11. Soybean germ concentrate comprising more than 85 % by weight of soybean germs, of which at least 80%, and preferably at least 90% by weight thereof are intact germs which have not been broken.

12. Soybean germ concentrate according to claim 11 obtainable by carrying out the method of any of claims 1 to 6.

13. Soybean germ oil obtainable from a soybean germ concentrate according to claim 11 or 12, and/or obtainable by carrying out the method of claim 7, having an amount of soybean phytosterols of at least 6%, and preferably of at least 7% by weight.

14. Soybean germ oil according to claim 13, having an amount of linolenic acid (C18:3) of between 10 and 30 wt%, preferably between 12 and 28 wt%, more preferably between 15 and 25 wt%, most preferably between 18 and 23 wt% on total fatty acids wt%.

15. Soybean germ oil according to claim 13 or 14, having an amount of soybean tocopherols of at least 0.10%, more preferably at least 0.20%, most preferably at least 0.25% by weight.

16. Soybean germ oil according to any of claims 13 to 15, substantially free of lecithin.

17. Use of a soybean germ concentrate according to claims 11 or 12 in a food product, a food supplement, a pharmaceutical composition or a cosmetic product.

18. Use of a soybean germ oil according to any of claims 13 to 16 in a food product, a food supplement, a pharmaceutical composition or a cosmetic product.

19. Food product comprising a soybean germ concentrate according to claims 11 or 12, wherein at least 80%, and preferably at least 90% by weight of the soybean germs in the concentrate are intact germs which have not been broken.

## Patentansprüche

1. Verfahren zum Verarbeiten ganzer Sojabohnen, welches die folgenden Schritte aufweist:
a) Aufbrechen ganzer Sojabohnen in einem Sojabohnenprodukt (2), das Keimblätter, Keimblattstücke, Schalen und Keime aufweist,
b) Anreichern eines Sojabohnenmaterials (5) aus dem Sojabohnenprodukt (2), das im Wesentlichen Sojabohnenkeimblattstücke und Sojabohnenkeime aufweist, und
c) Trennen der Sojabohnenkeime (6) von dem Sojabohnenmaterial (5), wobei die Trennung auf einem Unterschied der Form zwischen den Sojabohnenkeimen und den Sojabohnenkeimblattstücken beruht und wobei der Trennschritt mithilfe einer gekerbten zylindrischen Vorrichtung durchgeführt wird.

2. Verfahren nach Anspruch 1, wobei das Sojabohnenmaterial (5) ausgehend von der Gesamtmenge des Materials mehr als 80 Gew.-% Sojabohnenkeimblattstücke und Sojabohnenkeime aufweist.

3. Verfahren nach Anspruch 1 oder 2, wobei das Sojabohnenmaterial (5) mit den folgenden Schritten erhalten wird:
a1) Trennen des Sojabohnenproduktes (2) durch mittelstarke Aspiration (11a) zu einer Schalenfraktion (3a), die im Wesentlichen Schalen aufweist, und einer entschalten Fraktion (4a), die im Wesentlichen Keimblätter, Keimblattstücke und Keime aufweist, und
a2) Anreichern des Sojabohnenmaterials (5) aus der entschalten Fraktion (4a) mit mindestens einem Sieb- und/oder Aspirationsschritt.

4. Verfahren nach Anspruch 3, welches des Weiteren ein Anreichern des Sojabohnenmaterials (5) aus der Schalenfraktion (3a) mit mindestens einem Sieb- und/oder Aspirationsschritt aufweist.

5. Verfahren nach Anspruch 1 oder 2, wobei das Sojabohnenmaterial (5) mit den folgenden Schritten erhalten wird:
b1) Trennen des Sojabohnenproduktes (2) durch starke Aspiration (11b) zu einer Schalenfraktion (3b), die im Wesentlichen Schalen, Keime und Keimblattstücke aufweist, und einer entschalten Fraktion (4b), die im Wesentlichen Keimblätter und Keimblattstücke Keime aufweist, und
b2) Anreichern des Sojabohnenmaterials (5) aus der Schalenfraktion (3b) mit mindestens einem Sieb- und/oder Aspirationsschritt.

6. Verfahren nach einem der Ansprüche 1 bis 5, wobei die ganzen Sojabohnen vor dem Aufbrechen zwischen 1 und 10 Min. lang auf eine Temperatur zwischen 60 °C und 80 °C erhitzt werden.

7. Verfahren nach einem der Ansprüche 1 bis 6, welches des Weiteren den Schritt des Unterziehens der Sojabohnenkeine (6) einer superkritischen CO₂-Extraktion und des Zubereitens von Sojabohnenkeimöl und Sojabohnenkeimmehl aus den extrahierten Sojabohnenkeimen aufweist.

8. Verwendung einer gekerbten zylindrischen Vorrichtung zum Trennen von Sojabohnenmaterial zu einem Sojabohnenhypokotylkonzentrat und Sojabohnenkeimblattstücken.

9. Verwendung einer gekerbten zylindrischen Vorrichtung nach Anspruch 8, wobei die Zylindertrommel der gekerbten zylindrischen Vorrichtung einen zylindrischen Mantel aufweist, der eine Form und eine Gestalt aufweist, um vorübergehend Sojabohnenhypokotyle aufzunehmen.

10. Verwendung einer gekerbten zylindrischen Vorrichtung nach Anspruch 8 oder 9, wobei das Sojabohnenmaterial einer Zentrifugalkraft von mindestens 0,1-mal Schwerkraft, vorzugsweise von mindestens 0,2-mal Schwerkraft, unterzogen wird.

11. Sojabohnenkeimkonzentrat, welches mehr als 85 Gew.-% Sojabohnenkeime aufweist, von denen mindestens 80 Gew.-% und vorzugsweise mindestens 90 Gew.-% intakte Keime sind, die nicht aufgebrochen wurden.

12. Sojabohnenkeimkonzentrat nach Anspruch 11, das durch Ausführen des Verfahrens nach einem der Ansprüche 1 bis 6 erhalten werden kann.

13. Sojabohnenkeimöl, das aus einem Sojabohnenkeimkonzentrat nach Anspruch 11 oder 12 erhalten werden kann und/oder durch Ausführen des Verfahrens von Anspruch 7 erhalten werden kann, das eine Menge an Sojabohnen-Pflanzensterolen von mindestens 6 Gew.-% und vorzugsweise von mindestens 7 Gew.-% aufweist.

14. Sojabohnenkeimöl nach Anspruch 13, das eine Menge an Linolensäure (C18:3) zwischen 10 Gew.-% und 30 Gew.-%, vorzugsweise zwischen 12 Gew.-% und 28 Gew.-%, insbesondere zwischen 15 Gew.-% und 25 Gew.-%, am meisten bevorzugt zwischen 18 Gew.-% und 23 Gew.-% des Gesamtgewichtsanteils an Fettsäuren aufweist.

15. Sojabohnenkeimöl nach Anspruch 13 oder 14, das eine Menge an Sojabohnen-Tocopherolen von mindestens 0,10 Gew.-%, insbesondere von mindestens 0,20 Gew.-%, am meisten bevorzugt von mindestens 0,25 Gew.-% aufweist.

16. Sojabohnenkeimöl nach einem der Ansprüche 13 bis 15, das im Wesentlichen frei von Lecithin ist.

17. Verwendung eines Sojabohnenkeimkonzentrats nach Anspruch 11 oder 12 in einem Nahrungsmittelprodukt, einem Nahrungsergänzungsmittel, einer pharmazeutischen Zusammensetzung oder einem kosmetischen Produkt.

18. Verwendung eines Sojabohnenkeimöls nach einem der Ansprüche 13 bis 16 in einem Nahrungsmittelprodukt, einem Nahrungsergänzungsmittel, einer pharmazeutischen Zusammensetzung oder einem kosmetischen Produkt.

19. Nahrungsmittelprodukt, das ein Sojabohnenkeimkonzentrat nach Anspruch 11 oder 12 aufweist, wobei mindestens 80 Gew.-% und vorzugsweise mindestens 90 Gew.-% der Sojabohnenkeime in dem Konzentrat intakte Keime sind, die nicht aufgebrochen wurden.

## Revendications

1. Procédé de transformation de grains de soja entiers, qui comprend les étapes consistant à :
a) broyer les grains de soja entiers en un produit de soja (2) comprenant des cotylédons, des déchets, des coques et des germes,
b) enrichir à partir dudit produit de soja (2) un matériau de soja (5) comprenant essentiellement des déchets de soja et des germes de soja, et
c) séparer lesdits germes de soja (6) dudit matériau de soja (5), ladite séparation étant basée sur une différence de forme entre lesdits germes de soja et lesdits déchets de soja, et ladite étape de séparation étant réalisée au moyen d'un dispositif cylindrique à indentation.

2. Procédé selon la revendication 1, dans lequel ledit matériau de soja (5) comprend plus de 80 % en poids de déchets de soja et de germes de soja par rapport à la quantité totale dudit matériau.

3. Procédé selon la revendication 1 ou 2, dans lequel ledit matériau de soja (5) et obtenu par les étapes consistant à :
a1) séparer par aspiration modérée (11a) ledit produit de soja (2) en une fraction de coques (3a) comprenant essentiellement des coques et une fraction décortiquée (4a) comprenant essentiellement des cotylédons, des déchets et des germes, et
a2) enrichir à partir de ladite fraction décortiquée (4a) ledit matériau de soja (5) par au moins une étape de sassage et/ou d'aspiration.

4. Procédé selon la revendication 3, comprenant en outre l'enrichissement à partir de ladite fraction de coques (3a) dudit matériau de soja (5) par au moins une étape de sassage et/ou d'aspiration.

5. Procédé selon la revendication 1 ou 2, dans lequel ledit matériau de soja (5) est obtenu par les étapes consistant à :
b1) séparer par aspiration forte (11b) ledit produit de soja (2) en une fraction de coques (3b) comprenant des coques, des germes et des déchets et une fraction décortiquée (4b) comprenant essentiellement des cotylédons et des déchets, et
b2) enrichir à partir de ladite fraction de coques (3b) ledit matériau de soja (5) par au moins une étape de sassage et/ou d'aspiration.

6. Procédé selon l'une quelconque des revendications 1 à 5, dans lequel lesdits grains de soja entiers sont chauffés à une température dans la plage de 60 à 80°C pendant 1 à 10 minutes avant d'être broyés.

7. Procédé selon l'une quelconque des revendications 1 à 6, comprenant en outre l'étape consistant à soumettre lesdits germes de soja (6) à une extraction au CO₂ supercritique et à préparer de l'huile de soja et de la farine de soja à partir desdits germes de soja extraits.

8. Utilisation d'un dispositif cylindrique à indentation pour séparer le matériau de soja en un concentré d'hypocotyles de soja et des déchets de soja.

9. Utilisation d'un dispositif cylindrique à indentation selon la revendication 8, dans lequel le tambour cylindrique dudit dispositif cylindrique à indentation comprend une enveloppe cylindrique ayant une forme et une configuration permettant de temporairement retenir les hypocotyles de soja.

10. Utilisation d'un dispositif cylindrique à indentation selon la revendication 8 ou 9, dans lequel ledit matériau de soja est soumis à une force centrifuge d'une gravité d'au moins 0,1 fois, de préférence d'une gravité d'au moins 0,2 fois.

11. Concentré de germes de soja comprenant plus de 85 % en poids de germes de soja, dont au moins 80 % et de préférence 90 % en poids sont des germes intacts qui n'ont pas été broyés.

12. Concentré de germes de soja selon la revendication 11 pouvant être obtenu en réalisant le procédé selon l'une quelconque des revendications 1 à 6.

13. Huile de soja pouvant être obtenue à partir d'un concentré de germes de soja selon la revendication 11 ou 12, et/ou pouvant être obtenue en réalisant le procédé selon la revendication 7, ayant une quantité de phytostérols de soja d'au moins 6 %, et de préférence d'au moins 7 % en poids.

14. Huile de soja selon la revendication 13, ayant une quantité d'acide linolénique (C18:3) de 10 à 30 % en poids, de préférence de 12 à 28 % en poids, de manière davantage préférée de 15 à 25 % en poids, et idéalement de 18 à 23 % en poids par rapport au pourcentage en poids total d'acides gras.

15. Huile de soja selon la revendication 13 ou 14, ayant une quantité de tocophérols de soja d'au moins 0,10 %, de préférence d'au moins 0,20 %, idéalement d'au moins 0,25 % en poids.

16. Huile de soja selon l'une quelconque des revendications 13 à 15, sensiblement dépourvue de lécithine.

17. Utilisation d'un concentré de germes de soja selon les revendications 11 ou 12 dans un produit alimentaire, un supplément alimentaire, une composition pharmaceutique ou un produit cosmétique.

18. Utilisation d'une huile de soja selon l'une quelconque des revendications 13 à 16 dans un produit alimentaire, un supplément alimentaire, une composition pharmaceutique ou un produit cosmétique.

19. Produit alimentaire comprenant un concentré de germes de soja selon les revendications 11 ou 12, dans lequel au moins 80 % et de préférence au moins 90 % en poids des germes de soja dans le concentré sont des germes intacts qui n'ont pas été broyés.
